# EUROPÄISCHE PATENTSCHRIFT

(11) **EP 2 838 580 B1**
(45) Veröffentlichungstag und Bekanntmachung des Hinweises auf die Patenterteilung: **21.03.2018**
(21) Anmeldenummer: 13720233.9
(22) Anmeldetag: 17.04.2013
(51) Int. Cl.: A61M 1/16, A61J 1/10, A61J 1/20

(54) **BEUTEL MIT STRÖMUNGSAUSGLEICH**
BAG WITH FLOW EQUALISATION
POCHE À COMPENSATION D'ÉCOULEMENT

(30) Priorität: 19.04.2012 DE 102012007697; 19.04.2012 US 201261635583 P
(43) Veröffentlichungstag der Anmeldung: 25.02.2015
(73) Patentinhaber: Fresenius Medical Care Deutschland GmbH, 61352 Bad Homburg (DE)
(72) Erfinder: KUGELMANN, Franz, 66606 St. Wendel/Bliesen (DE); HAUPERT, Claus, 66809 Nalbach (DE); HÖRMANN, Jörn, 66265 Heusweiler (DE)
(74) Vertreter: Herrmann, Uwe
(86) Internationale Anmeldenummer: PCT/EP2013/001128
(87) Internationale Veröffentlichungsnummer: WO 2013/156150

(56) Entgegenhaltungen:
- EP-A2- 0 004 600
- WO-A1-97/37628
- WO-A1-2011/073274
- DE-A1- 19 510 759
- US-A- 4 767 526

## Beschreibung

Die Erfindung betrifft das Gebiet der Bereitstellung und Speicherung von gebrauchsfertiger Flüssigkeit für die Nierentherapie. Insbesondere betrifft die Erfindung ein Beutelsystem zur Herstellung und Aufnahme von frischer und gebrauchter Dialysierflüssigkeit. Weiterhin ist Gegenstand der Erfindung ein Mehrkammerbeutel zur Aufnahme von Konzentraten und Herstellung einer medizinischen Lösung aus den Konzentraten, sowie ein Verfahren zur Herstellung einer medizinischen Lösung aus mehreren Konzentraten.

Niereninsuffiziente Patienten leiden unter eingeschränkter Funktion der Nieren, so dass eine notwendige Ausscheidung von harnpflichtigen Substanzen aus dem Körper des Patienten verhindert wird. Die Abscheidung von toxischen Metaboliten aus dem Patienten muss durch Blutreinigung in einer Dialysebehandlung erfolgen.

In der Dialyse erfolgt eine Blutreinigung über eine Stoffaustauschmembran, die auf der einen Seite mit dem Patientenblut und auf der anderen Seite mit einer Reinigungsflüssigkeit in Kontakt kommt. Bei der Reinigungsflüssigkeit handelt es sich um sogenannte Dialyselösungen, die die zur Ausscheidung bestimmten Substanzen aufnimmt und aus dem Patientenblut abtransportiert. Im Allgemeinen bestehen Dialyselösungen aus einer wässrigen Zusammensetzung von physiologisch wichtigen gelösten Komponenten, zum Beispiel Elektrolyten, Puffern und osmotisch aktiven Reagenzien, zum Beispiel Glukose.

In der Peritoneal Dialyse (PD) wird die Dialyselösung in das Peritoneum des Patienten infundiert. Das Bauchfell des Patienten dient dabei als Stoffaustauschmembran, durch die eine Reinigung des Blutes erfolgt. Im Laufe der Therapie oder gegen Ende der Therapie wird das Peritoneum entleert und die Dialyselösung verworfen.

In der Hämodialyse (HD) wird das zu reinigende Blut des Patienten durch einen extrakorporalen Blutkreislauf geführt und mit einer künstlichen Stoffaustauschmembran in Kontakt gebracht. Die gegenüberliegende Seite der Stoffaustauschmembran wird mit Dialyselösung in Kontakt gebracht, so dass membrangängige Substanzen aus dem Blut durch Membranübergang mit der Dialyselösung abtransportiert werden können.

In beiden Fällen der HD und PD Dialyse enthalten die Dialyselösungen typische gelöste Stoffe wie zum Beispiel:
- Elektrolyte Na, K, Mg, Ca, um einen annehmbaren Elektrolythaushalt des Patienten aufrecht zu erhalten
- Puffer (zum Beispiel Bikarbonat, Acetat, Laktat usw.)
- Glukose (oder andere osmotische Agenzien), als osmotisches Mittel in der Peritonealdialyse oder zur Aufrechterhaltung des Blutzuckspiegels während der Hämodialyse
- Säuren oder Salze von Säuren (zum Beispiel HCl bzw. Chlorid, Essigsäure bzw. Acetat, Zitronensäure bzw. Citrat), die eventuell zur Neutralisation basischer Teil-Dialyselösungen beitragen oder als Gegenionen im elektrochemischen Gleichgewicht vorliegen

In der Hämodialyse werden die Teillösungen oder Teilkonzentrate oftmals in der Dialysemaschine vor und während des Verlaufs der Behandlung gemischt und zu einer fertigen Dialyselösung hergestellt. Hierfür werden oft Teilkonzentrate in fester oder in flüssiger Form verwendet, die in einzelnen Behältern vorliegen und durch einen Anschluss an die Dialysemaschine mithilfe eines vorbereiteten Hydrauliksystems verdünnt, gemischt und zur fertigen verwendbaren Dialyselösung aufbereitet werden.

In einer weiteren Variante werden Dialyselösungen in einem Batch in einem der Behandlung vorgelagerten Schritt aufbereitet. Das Batch wird in einem Tank bevorratet, der dafür vorbereitet ist, mit einer Dialysemaschine verbunden zu werden. In manchen Fällen ist der Tank ein integraler Bestandteil einer Dialysebehandlungseinheit. In anderen Ausgestaltungen ist der Dialysetank von der Behandlungsstation separat und funktional beweglich ausgestaltet. Die Verwendung eines Tanks mit Dialysierflüssigkeit kann somit den Vorteil aufweisen, durch eine einmalige Zubereitung der Dialysierflüssigkeit den Behandlungsplatz relativ ortsunabhängig zu wählen. Somit sind Behandlungsstationen an verschiedenen Orten einsetzbar, ohne auf eine Zubereitungseinheit von Dialyselösung oder einem Wasseranschluss, der das notwendige Wasser zur Verdünnung der Konzentrate liefert, angewiesen zu sein. In diesen Fällen wird die Dialyselösung an einer dafür vorgesehenen Einrichtung aus Konzentraten zusammengemischt und in einem meist mobilen Tank bevorratet.

Darüber hinaus sind Containersysteme bekannt, deren gebrauchsfertige Lösung in einem Behältnis vorgelegt wird und dieses Behältnis z.B. als innerer Beutel von einem weiteren Container umschlossen ist. Aus der Getränketechnologie sind vor allem solche Konzepte bekannt und werden unter anderem als "bag in box" Systeme beschrieben.

Weiter entwickelte Systeme im Bereich der Dialysetherapie stellen Systeme dar, bei denen das innere Behältnis die gebrauchsfertige Flüssigkeit beinhaltet und der das innere Behältnis umgebende Container dafür vorbereitet ist, die gebrauchte Flüssigkeit aufzunehmen. Das innere Behältnis wird dann von der gebrauchten Flüssigkeit im äußeren Container umgeben.

US 4,386,634 zeigt ein Behältersystem zur Zubereitung einer gebrauchsfertigen Lösung für die Hämodialyse. In einem inneren großvolumigen Folienbeutel wird die gebrauchsfertige Lösung durch Auflösen von Konzentraten hergestellt. Der innere Beutel ist von Wasser im äußeren Container umgeben.

DE 195 10 759 zeigt ein Containersystem bestehend aus einem inneren großvolumigen Folienbeutel und einem biegsteifen, den inneren Beutel umgebenden Container. Die gebrauchsfertige Dialyseflüssigkeit wird in dem inneren Beutel vorgelegt und die gebrauchte Flüssigkeit wird in den Raum zwischen innerem Beutel und äußerem umgebenden Container gefüllt.

WO 2011/073274 zeigt ein Beutelsystem mit einem inneren Beutel und einem den inneren Beutel umgebenden äußeren Beutel. Die Dialysierflüssigkeit wird im inneren Beutel durch Auflösen der Konzentrate hergestellt. Gebrauchte Dialysierflüssigkeit wird in den Raum zwischen innerem Beutel und äußerem Beutel gefüllt. Das ganze Beutelsystem kann zusätzlich in einer biegesteifen Stützapparatur eingelegt werden.

In den im Stand der Technik beschriebenen Containersystemen kann der innere flexible Behälter durch Faltenbildung oder durch eine nicht fixierte Position im Raum des umgebenden Containers die Strömungsbedingungen im äußeren Container ungünstig beeinflussen. Insbesondere kann der innere Beutel den Zuströmungsbereich des äußeren Containers verdecken, so dass zuströmende gebrauchte Flüssigkeit daran gehindert ist, in den äußeren Container einzutreten. Weiterhin kann der bewegliche innere Beutel verhindern, dass sich zuströmende gebrauchte Flüssigkeit in dem äußeren Container gleichmäßig verteilen kann. Evtl. werden beim Zuströmen der gebrauchten Flüssigkeit zunächst nur Teile des äußeren Containers befüllt. Durch den anwachsenden hydrostatischen Druck kann der innere Beutle schlagartig verschoben werden und es kommt zu einem ebenfalls schlagartigen Strömungsausgleich im äußeren Container, der zu unzulässigen Erschütterungen der gesamten Apparatur und zu unzulässig hoher Materialbeanspruchung führen kann.

Eine erste Maßnahme, die vermeiden kann, dass es zu einer zu großen Beweglichkeit des inneren Beutels im äußeren Container kommt, beschreibt schon die WO 2011/073274. Darin ist gezeigt, die Ränder des inneren Beutels entlang einer Schweißlinie mit den Rändern des umgebenden Beutels zu verschweißen. Der innere Beutel wird dadurch umfänglich in dem äußeren Beutel fixiert. Jedoch zeigt sich, dass auch diese Maßnahme nicht ausreichend ist, um eine gleichmäßige Befüllung des äußeren Containers zu erreichen.

Aufgabe war es daher, ein Containersystem für die Herstellung und/oder Aufbewahrung von gebrauchsfertigen Flüssigkeiten und/oder gebrauchten Flüssigkeiten mit einem inneren Beutel und einem den inneren Beutel umgebenden Container so weiterzubilden, dass bei Zuführung von Flüssigkeit in den äußeren Container ein guter Strömungsausgleich geschaffen wird, so dass der Container mit zunehmender Befüllung gleichmäßig gefüllt wird.

### Beschreibung der Erfindung

Der Erfindung liegt die Aufgabe zugrunde ein Containersystem bestehend aus einem inneren Container und einem äußeren, den inneren Container umgebenden Container, für die Aufnahme, Bereitstellung oder Herstellung von medizinischen Flüssigkeiten, insbesondere Flüssigkeiten für die extrakorporale Blutbehandlung oder die Nierentherapie, so weiterzubilden, dass eine gleichmäßige Befüllung des äußeren Containers gewährleistet ist.

Die Aufgabe wird durch den Gegenstand von Anspruch 1, einem Containersystem zur Herstellung, Bereitstellung und Aufnahme gebrauchsfertiger und/oder gebrauchter medizinischer Flüssigkeiten, insbesondere gebrauchsfertiger und gebrauchter Flüssigkeiten, wie sie in der extrakorporalen Blutbehandlung oder der Nierentherapie vorkommen, gelöst. Vorteilhafte Ausführungsformen werden durch die Merkmale der Unteransprüche dargestellt.

Das erfindungsgemäße Containersystem wird durch einen inneren Container und einen den inneren Container umgebenden äußeren Container gebildet. Innerer und äußerer Container bilden jeweils ein abgeschlossenes Volumen, so dass sie für die Aufnahme von Flüssigkeiten vorbereitet sind. An den jeweiligen Containern befinden sich Zugangsports, über die die Container mit Flüssigkeit befüllt werden können oder über die Flüssigkeit dem jeweiligen Container entnommen werden kann. Bei der Flüssigkeit kann es sich um eine Verdünnungsflüssigkeit handeln, also Wasser oder wässrige Lösungen von Elektrolyten und/oder Osmotika und/oder Wirkstoffen. Es kann sich bei den Lösungen auch um für die Nierentherapie gebrauchsfertig vorbereitete Lösungen handeln. Insbesondere sind Flüssigkeiten umfasst, die für die Peritonealdialyse, Hämodialyse, Hämofiltration, Hämodiafiltration und weitere extrakorporale Nierenunterstützende Therapieverfahren eingesetzt werden.

Der von dem äußeren Container umgebene innere Container weist weiterhin eine oder mehrere Durchgangsöffnungen auf, die Teile des Volumens des äußeren Beutels miteinander in Strömungsverbindung bringen. Der Begriff Container beschränkt sich dabei nicht nur auf den einer Flüssigkeit zugänglichen Innenraumbereich eines Behälters. Teile des Containers, die z.B. durch Abschweißung nicht zum Innenraum gehören und einer Flüssigkeit nicht zugänglich sind, werden ebenfalls in dieser Definition von dem Begriff Container umfasst. Die Durchgangsöffnungen können also auch in Bereichen des inneren Containers angeordnet sein, die für Flüssigkeit im inneren Container nicht zugänglich sind, die aber gleichwohl eine Strömungsbarriere für Flüssigkeiten im umgebenden Container bedeuten würden. Unter solchen Durchgangsöffnungen können rohrförmige Bauteile verstanden werden, z.B. aus einem biegesteifen Spritzgussmaterial, einem biegsamen Schlauchstück oder einem kollabierbaren Folienschlauchstück. Bevorzugt sind jedoch einfache, z.B. kreisrunde Öffnungen in gegenüberliegenden Bereichen des Containermaterials des inneren Containers. Durch Schweißung entlang der kreisrunden Kontur wird das innere Volumen des inneren Beutels gegenüber dem äußeren Beutel abgegrenzt und geschlossen. Flüssigkeit kann jedoch durch die Durchgangsöffnungen des inneren Beutels von einem Teil des äußeren Beutels in einen weiteren, z.B. gegenüberliegenden Teil des äußeren Containers strömen. Der innere Container stellt somit gegenüber einem evtl. geforderten Strömungsausgleich im äußeren Container eine geringere Barriere da. Befüll- und Entnahmevorgänge von Flüssigkeit werden daher verbessert.

Es können eine oder mehrere Durchgangsöffnungen für das Containersystem vorgesehen sein. Dies ist auch von dem zu erzielenden Gesamt-Volumen des inneren und/oder äußeren Containers abhängig. Insbesondere kann bei kleinen Volumen von z.B. 5 Litern des Containersystems nur eine Durchgangsöffnung vorgesehen sein. Bei größeren Volumen von z.B. 120 Litern wird ein Strömungsausgleich durch nur eine Durchgangsöffnung unsicher und es ist daher eine Vielzahl solcher Durchgangsöffnungen vorzusehen. Der erfinderische Gegenstand kann in bestimmten Ausführungen an Containersystemen mit Volumina von 60 Liter ± 20 Liter Anwendung finden.

In bevorzugten Ausführungsformen sind die Container als Beutel ausgestaltet. Ein Containersystem kann z.B. aus einem äußeren biegesteifen Container und einem inneren Container aus wenigstens abschnittsweise flexiblem Folienmaterial bestehen, der einen Beutel darstellt. Alternativ kann der innere Container aus biegesteifem Material bestehen und der äußere Container besteht wenigstens abschnittsweise aus flexiblem Folienmaterial und bildet einen Beutel. In einer weiteren bevorzugten Ausführungsform bestehen innerer und äußerer Container wenigstens abschnittsweise aus flexiblem Folienmaterial und bilden jeweils einen Beutel.

Unter Beutel sind in diesem Zusammenhang Behältnisse zu verstehen, die aus flexiblen Begrenzungsflächen bestehen, so dass sie in einem ungefüllten Zustand in einer kollabierten Form vorliegen und in einem befüllten Zustand entfaltet vorliegen. Dabei sind solche Behältnisse auch im Sinne dieses Begriffs umfasst, deren Begrenzungsflächen abschnittsweise aus einem biegesteifen Material bestehen, insgesamt aber durch flexible Begrenzungsflächen einen kollabierbaren Charakter aufweisen.

In einer Ausführungsform befindet sich der innere Beutel dabei in einem durch den äußeren Beutel definierten Volumen. Es ist dabei von Vorteil, wenn der innere Beutel an Befestigungspunkten oder Linien mit dem äußeren Beutel verbunden ist. Dadurch kann verhindert werden, dass der innere Beutel z.B. im Leerzustand unerwünscht gefaltet wird und diese Faltungen das Befüllen des inneren Beutels mit Flüssigkeit erschweren oder unmöglich machen. Im ungünstigsten Fall kann sich der innere Beutel nicht entfalten und es wird nur ein Minimum des zu erreichenden Füllvolumens erreicht. Die Befestigung des inneren Beutels mit dem äußeren Beutel kann z.B. durch Folienstücke erfolgen, die durch Schweißstellen den äußeren und den inneren Beutel verbinden. Alternativ kann vorgesehen sein, dass innerer Beutel und äußerer Beutel durch Schweißlinien miteinander verbunden sind. Diese können z.B. die umfänglichen Schweißnähte des Beutels sein. Alternativ kann auch eine erste Folienbahn des äußeren Beutels mit einer ersten Folienbahn des inneren Beutels entweder permanent oder peelbar (lösbar) verschweißt sein.

Unter peelbaren Schweißverbindungen sind in diesem Zusammenhang Verbindungsstellen zweier Fügepartner zu verstehen, die nach Wärmebehandlung und Anpressdruck eine Adhäsivwirkung aufeinander ausüben. Im vorliegenden Fall handelt es sich bei den Fügepartnern um zwei gegenüberliegende Folienstücke eines Beutels, die im Schweißprozess mit einem Schweißbalken durch Wärmeeinwirkung und Anpressdruck miteinander verbunden werden. Die Schweißtemperatur bedingt, mit welcher Kraft die Peelnaht geöffnet werden kann. Unter einer Peelnaht ist eine Adhäsivverbindung zu verstehen, die durch Kraftwirkung wieder gelöst werden kann, ohne dass ein vollständiger Bruch des Folienmaterials erfolgt. In besonderen Ausführungen kann unter Peelverbindung auch eine solche Verbindung verstanden werden, die durch Krafteinwirkung eine teilweise Delamination eines mehrschichtigen Folienverbundes verursacht. In diesen Fällen ist es wichtig, das der Delaminationsriss keinen vollständigen Bruch des Folienmaterials verursacht, was den Beutel unbrauchbar machen würde.

Um den Befüllvorgang oder Entnahmevorgang von Flüssigkeit weiterhin zu erleichtern, sind an dem jeweiligen inneren und äußeren Beutel Belüftungsports angebracht. Diese Ports enthalten eine hydrophobe Membran, deren Porengröße so gering ist, dass der Transport von Mikroorganismen oder Toxinen über die Membran hinweg ausgeschlossen ist. Das hydrophobe Membranmaterial ermöglicht eine ausreichende Sperrwirkung bei zufälligem Kontakt mit Dialysierflüssigkeiten, so dass keine Flüssigkeit nach außen dringen kann. Insbesondere kann beim Befüllen des inneren Beutels mit einer Verdünnungsflüssigkeit die darin enthaltene Luft durch das Zuströmen der Verdünnungsflüssigkeit über den Port entweichen. Insbesondere wird durch das Ausdehnen des inneren Beutels auch die Luft aus dem äußeren Beutel ausgedrückt. Alternativ wird auch beim Befüllen des äußeren Beutels durch bereits gebrauchte Dialyselösung Restluft aus dem äußeren Beutel über die Entlüftungsmembran entweichen. Sofern das Containersystem biegesteife Materialien umfasst sind Entlüftungsports unerlässlich. Im äußersten Fall sind solche Containersysteme nicht kollabierbar und es muss entsprechend bei der Befüllung und Entnahme von Flüssigkeiten zum Druckausgleich eine entsprechende Menge Luft oder ein alternatives Gas nachgeliefert werden.

Das Containersystem aus flexiblem Folienmaterial kann aus vier übereinander angeordneten Folien hergestellt sein. Dabei werden die Folien durch eine umlaufende Begrenzungslinie fixiert. Die Begrenzungslinie kann abschnittsweise oder vollständig aus einer Schweißverbindung, z.B. einer oder mehrerer Schweißnähte gebildet werden. An einer oberen Seite des Beutels kann die Schweißverbindung z.B. an eine zusätzliche biegesteife Kunststoffschiene erfolgen, die als Halterung für das Beutelsystem dient. Die seitlichen Begrenzungslinien und eine Begrenzungslinie am unteren Ende des Beutels können durch Schweißnähte gebildet werden. Insbesondere für den Fall, dass der innere und äußere Beutel aus flach gelegten Folienschläuchen gebildet werden, sind die seitlichen Schweißnähte nicht zwingend notwendig. Die seitlichen Begrenzungslinien werden sodann durch die Folienfalte des flach gelegten Folienschlauches gebildet. Die seitlichen Schweißnähte sind aber bevorzugt, da die Schweißnaht die vier übereinanderliegenden Folien des Beutelsystems verschweißen und den inneren Beutel damit im äußeren Beutel zusätzlich fixiert.

In einer derartigen Ausgestaltung der umlaufenden Verschweißung der Folien würde das Beutelsystem aus einer ersten Kammer des inneren Beutels und zwei weiteren Kammern des äußeren Beutels, die auf den jeweils angrenzenden Seiten des inneren Beutels angeordnet sind, aufgebaut sein. Die äußeren Kammern des äußeren Beutels stehen durch die Durchgangsöffnungen in Strömungsverbindung miteinander. Wird der äußere Beutel entsprechend den äußeren Kammern mit z.B. gebrauchtem Dialysat befüllt, so kann ein Strömungsausgleich über die Öffnungen stattfinden und der Beutel gleichmäßig befüllt werden, ohne dass unzulässige Materialbeanspruchungen entstehen.

Dabei kann vorgesehen sein, dass die inneren oder die äußeren Kammern weitere Kompartimente enthalten, die für die Herstellung der Dialysierflüssigkeit mit Konzentraten befüllt sind. Solche Kompartimente können z.B. durch Peelschweißverbindungen gegenüberliegender Folienabschnitte gebildet werden. Insbesondere werden die gegenüberliegenden Folien des inneren Beutels peelbar verschweißt, so dass bei Befüllung des inneren Beutels mit z.B. Verdünnungsflüssigkeit die Kompartimente aufgebrochen und die Konzentrate zur gebrauchsfertigen Dialysierflüssigkeit verdünnt werden. Die Konzentrate können dabei in pulverförmiger, flüssiger Form oder als Dispersion vorliegen.

Das bisher beschriebene Containersystem eignet sich insbesondere für ein Verfahren zur Herstellung, Bereitstellung oder Aufnahme einer physiologischen Flüssigkeit. Dabei wird eine Flüssigkeit für die Nierentherapie durch Befüllung des inneren Containers des zuvor beschriebenen Containersystems hergestellt. In diesem Verfahrensschritt strömt Verdünnungsflüssigkeit in das Innere des inneren Beutels und bricht durch den sich aufbauenden Fülldruck ein oder mehrere Kompartimente auf, die mit Konzentraten befüllt sind. In dem weiter ansteigenden Flüssigkeitsvolumen durch Zuströmen des Verdünnungsmittels lösen sich die freigesetzten Konzentrate auf und bilden die gebrauchsfertige Dialysierflüssigkeit. In einem weiteren Schritt wird dem inneren Beutel durch einen Port Flüssigkeit zur weiteren Verwendung entnommen. Dabei kann die Flüssigkeit für Dialysiervorgänge eines Dialysepatienten verwendet werden, z.B. in der Peritonealdialyse oder in Dialysetherapien der extrakorporalen Blutbehandlung. Unter Dialysetherapien der extrakorporalen Blutbehandlung werden auch Blutfiltrationsverfahren, z.B. Hämofiltrations- oder Hämodiafiltrationsverfahren verstanden. Insgesamt werden darunter extrakorporale Bluttherapien verstanden, bei denen Bestandteile des Blutes über einen Filter abgetrennt werden und das Blut in Austauschbeziehung mit einer physiologischen Flüssigkeit steht, entweder über einen transmembranen Transport oder auch durch Infusion.

Nach erfolgter Nutzung der Flüssigkeit wird die gebrauchte Flüssigkeit dem äußeren Beutel Beutelsystems zurückgeführt. Die Kammern des äußeren Beutels werden aufgrund der vorhandenen Durchgangspassagen des inneren Beutels gleichmäßig durchströmt. Dabei wird gewährleistet, dass die zuströmende Flüssigkeit sich auf beide Seiten des inneren Beutels verteilt. Würde der äußere Beutel nur auf einer Seite befüllt werden können, weil der Strömungsausgleich zur zweiten Hälfte verhindert ist, entstünde ungewollt eine doppelte Belastung von Folien und Schweißnähten. Insbesondere hat sich für großvolumige Beutel die Verwendung von elastischem Folienmaterial bewährt, so dass der Beutel wie bei einem Luftballon unter Befüllung erst unter Dehnung der Folien sein endgültiges Zielvolumen erreicht. Bei solchen Beutelsystemen ist gerade die gleichmäßige Befüllung besonders von Bedeutung, um abschnittsweise ein Überdehnen der Folie und ein damit verbundenes Reißen der Folienwand zu verhindern.

### Detaillierte Beschreibung der Erfindung anhand der Zeichnungen

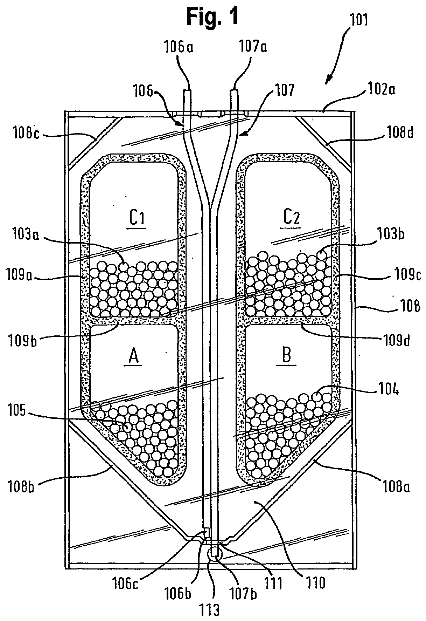

**Fig. 1** zeigt in schematischer Darstellung ein Containersystem ohne Durchgangsöffnungen. Das Containersystem besteht aus einer oberen Außenwandung 102a und einer unteren nicht gezeigten Außenwandung 102b, die durch einen gemeinsamen umfänglichen Rand 108 begrenzt und fluiddicht verbunden sind. In einer bevorzugten Version sind 102a und 102b die oberen und unteren Folienblätter eines Beutels, 108 wird durch eine permanente Schweißnaht gebildet.

**Tabelle 1**

| Komponente | Konzentratkammer | Einwaage [g] | Konzentration in gebrauchsfertiger Lösung [mmol/l] | Beitrag zur elektrischen Leitfähigkeit in Lösung |
|---|---|---|---|---|
| Basisches Magnesiumcarbonat 4MgCO₃ x Mg(OH)₂ x 5H₂O | A | 3,01 | 0,5 | Im Wesentlichen nicht vorhanden |
| D-Glukose wasserfrei | B | 62 | 5,55 | Im Wesentlichen nicht vorhanden |
| Calciumchlorid wasserfrei | A | 8,62 | 1,25 | Im Wesentlichen nicht vorhanden |
| Zitronensäure | A | 11,97 | 1,0 | Im Wesentlichen nicht vorhanden |
| Kochsalz | C | 391,22 | 140 | Vorhanden |
| Natriumhydrogencarbonat | C | 166,78 | 32 | Vorhanden |
| Kaliumchlorid | A | 28,1 | 2 | Im Wesentlichen nicht vorhanden |

Der Beutel verfügt über eine erste Konzentratkammer A mit Konzentraten 105. Nach den beispielhaften Ausführungen der Tabelle 1 befinden sich in der Konzentratkammer A das Konzentrat 105, die Substanzen Magnesiumcarbonat oder allgemein lösliche Magnesiumsalze, Calciumsalze, zum Beispiel gelöst oder in fester Form, Magnesiumchlorid und/oder Calciumchlorid, Zitronensäure oder Zitratsalze, Säuresalze oder Säuren in fester oder in gelöster Form.

Eine zweite Konzentratkammer C1 enthält ein weiteres Konzentrat 103a. In einer beispielhaften Ausführung kann es sich dabei um Natriumchlorid und/oder Bikarbonat oder allgemein um lösliche Salze der Kohlensäure, zum Beispiel Natriumcarbonat oder anderen physiologisch verträglichen Puffern, zum Beispiel Salze von schwachen Säuren handeln.

Die Konzentratkammern A und C1 werden entlang einer geschlossenen Umfassungslinie von einer Trennlinie 109a umschlossen, die in der dargestellten Ausführungsform aus einer wenigstens abschnittsweise semipermanenten Trennlinie besteht. In einer Ausführungsform besteht die erste Trennlinie aus 109a aus einer Peelnaht. Die Inhalte der Konzentratkammern A und C1 werden durch die weitere Trennlinie 109b voneinander getrennt. Die weitere Trennlinie 109b kann aus einer permanenten Schweißlinie, einer teilweise semipermanenten Schweißlinie oder einer Peelnaht bestehen. Vorzugsweise sind die Trennlinien 109a und 109b als Peelnähte ausgestaltet und bilden eine zusammenhängende integrale Konstruktion von Peelnahtabschnitten.

Die beispielhafte Ausführung nach **Fig. 1** ist weiterhin dadurch gekennzeichnet, dass ein Verbindungsport 106 die Füllkammer 110 des Beutels über ein erstes Ende im Inneren des Beutels 106b und einem weiteren Ende 106a außerhalb des Beutels 101 mit dem Äußeren des Beutels verbindet. Port 106 ist vorzugsweise durch Schweißverbindungen fluiddicht in der umfänglichen Trennlinie 108 befestigt. Vorzugsweise befindet sich am Ende 106b des Ports 106 ein Mittel 106c zur Erzeugung von Fluidturbulenzen des zuströmenden Verdünnungsmittels. Dieses Mittel kann als Turbulenz erzeugende Düse ausgestaltet sein oder als eine Turbulenz erzeugende Fritte. Weiterhin besteht Port 106 aus einem Schlauch, der den Beutel im Inneren in seiner länglichen Ausdehnung durchmisst. Es wird so im Befüllverfahren gewährleistet, dass bei aufrechter Lagerung des Beutels, zum Beispiel durch Aufnahme des Beutels an einer oberen Halteschiene 112, von unten befüllt wird und die Konzentratkammern A, B, C1, C2 in der Abfolge A gleichzeitig mit B, vor C1 gleichzeitig mit C2, durch den inneren Fülldruck geöffnet werden.

Ein weiterer Port 107 mit einem ersten Ende 107a außerhalb des Beutels und einem weiteren Ende 107b dient zur Rückführung verbrauchter medizinischer Flüssigkeit, bevorzugt Dialyselösung. Port 107 ist dabei im Inneren des Beutels als Schlauch ausgebildet und dafür vorgesehen, bei hängender Lagerung des Beutels, zum Beispiel durch Aufnahme des Beutels an der oberen Halteschiene 112, den Beutel entlang einer länglichen Ausdehnung zu durchmessen. An der Stelle 111 durchdringt der Schlauch 107 die Beutel-Umfassungslinie 108 des Beutels 101 und mündet in eine weitere, nicht gezeigte Kammer. 111 kann dabei eine fluiddichtende Schweißstelle sein, die den Beutel zwischen den oberen und unteren Begrenzungsebenen 102a und 102b fixiert und Teil der geschweißten Umfassungslinie 108 ist. Die nicht dargestellte Kammer ist ein umhüllender Container, bevorzugt Beutel, der integraler Bestandteil des Containersystems 101 ist. Es ergibt sich so eine "bag in bag" Konstruktion, bei der der Beutel, der die gebrauchsfertige Flüssigkeit aufnimmt, von einem Beutel, der die gebrauchte Flüssigkeit aufnimmt, umhüllt wird.

Weiterhin zeigt die Ausführung in **Fig. 1** einen zweiten Satz von Konzentratkammern B, C2, die durch eine weitere Trennlinie entlang einer geschlossenen Umfassungslinie 109c umschlossen sind. Eine weitere Trennlinie 109d trennt die Inhalte der Konzentratkammern B und C2. In einer bevorzugten Ausführungsform bilden die Peelnähte 109c und 109d eine integrale Konstruktion aus Peelnahtabschnitten, die ineinander übergehen.

In der beispielhaften Ausführungsform umfasst das Konzentrat 104 in Kammer B z.B. ein Glukosekonzentrat in pulverförmiger und/oder wasserfreier oder in flüssiger Form.

Eine weitere Konzentratkammer C2 mit Konzentrat 103b enthält weitere Substanzen, die mit den Substanzen der Konzentrate 105, 104 unverträglich sind, also zur Degradation neigen oder unerwünschte Wechselwirkung eingehen.

Die Ausführungsform zeigt weiterhin eine Umfassungslinie 108, die vorzugsweise aus einer permanenten Schweißnaht besteht. Zusätzliche permanente Schweißlinien, Abschnitte 108a und 108b, begrenzen den Containerinhalt oder Beutelinhalt so, dass ein schräger Boden des Innenraums entsteht. Der Begriff Container beschränkt sich dabei nicht nur auf den einer Flüssigkeit zugänglichen Innenraumbereich. Teile des Containers, die z.B. durch Abschweißung nicht zum Innenraum gehören und einer Flüssigkeit nicht zugänglich sind, werden ebenfalls in dieser Definition von dem Begriff Container umfasst. Die Konstruktion begünstigt die Verwirbelung des zuströmenden Verdünnungsmittels durch Fluidturbulenz erzeugendes Mittel 106c und damit den Lösungsprozess der Komponenten 105, 104, 103a, 103b. Begrenzungslinien 108c und 108d verleihen dem gefüllten Container, insbesondere Beutel, weitere Stabilität im gefüllten Zustand. Dies ist insbesondere für großvolumige Container von Bedeutung, bei denen der Innendruck durch die beinhalteten Mengen belastende Spannungswirkung auf die Umfassungslinie 108 ausüben kann. Großvolumige Beutel sind in diesem Sinne als Container zu verstehen, die ein Volumen von 5 bis 120 Liter, 40 bis 80 Liter, insbesondere 60 Liter ± 15% aufweisen.

In einer Ausführung wird die Füllkammer über den Port 106 unter Lösen der Konzentrate aus den Kammern A, B, C1, C2 gefüllt. Zur Entnahme der hergestellten Lösung wird über ein externes Pumpenmittel, wie z.B. eine Schlauchrollenpumpe oder eine Membranpumpe der Inhalt für die Dialysetherapie entnommen. Nach Gebrauch der entnommenen Flüssigkeit wird diese über den Port 107 in das Beutelsystem zurückgeführt. Der innere Beutel weist um das Portende 107b eine runde Aussparung 113 der gegenüberliegenden Folien auf, so dass das Portende in Fluidverbindung mit dem Volumen des äußeren Beutels steht. In einem ungünstigen Fall kann der äußere Beutel nur eine Kammer des äußeren Beutels füllen, wenn z.B. durch Faltenbildung der Folien der Durchgang zur anderen Kammer des äußeren Beutels versperrt bleibt. In dieser Konstellation der Fig. 1 wird eine Kammer des äußeren Beutels immer weiter gefüllt, während die andere Kammer ungefüllt bleibt, so dass die Folien der befüllten Kammer über ein zugelassenes Volumen hinaus befüllt wird und das Folienmaterial oder die Schweißlinien zerstört werden können.

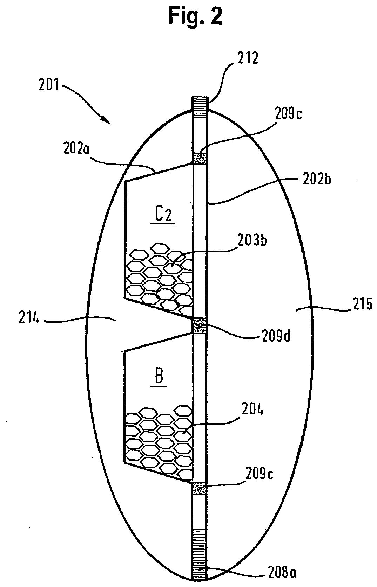

**Fig. 2** zeigt den Beutel in unbefülltem Zustand, also mit unversehrten Konzentratkammern in einer seitlichen Ansicht. Die seitliche Ansicht ist nur schematisch wiedergegeben und zeigt nicht alle Details aus der frontalen Ansicht aus **Fig. 1****.**

Das Beutelsystem wird demnach gebildet aus einem inneren Beutel mit einer Folienschicht 202a (entsprechend 102a) und einer gegenüberliegenden Folienschicht 202b (entsprechend 102b **Fig. 1**). Der innere Beutel ist durch umfängliche Schweißlinien (entsprechend 108 **Fig. 1**) gezeigt in dem äußeren Beutel befestigt. Die Schweißlinie 208 a bildet, wie in **Fig. 1** die Schweißlinie 108 a, den abgeschrägten Boden des inneren Beutels. Der innere Beutel trennt den äußeren Beutel in zwei Kammern 215 und 214. Weiterhin ist in **Fig. 2** schematisch der Portzugang 106 gezeigt, ebenso die Konzentratkammern C2 mit dem Konzentrat 203b (entspr. 103b), B mit Konzentrat 204 (entspr. 104). Weiterhin sind lösbare Verbindungen 209c gezeigt 209d, die bei Befüllung des inneren Beutels über den nicht gezeigten Port 106 aufgebrochen werden und die Konzentrate mit der zuströmenden Verdünnungsflüssigkeit vermischen lässt. Zum Gebrauch wird die Mischlösung des inneren Beutels entnommen, verwendet und über den nicht gezeigten Port 107 den Kammern 215 und 214 zur Aufbewahrung zugeführt. Aus **Fig. 2** ist ersichtlich, dass wenn nur eine Kammer des äußeren Beutels, z.B. 215, befüllt wird, weil, wie vorab beschrieben, der Zugang zur zweiten Kammer versperrt ist, diese Kammer unzulässig stark befüllt wird, was zu Materialermüdung des Beutelsystems führen kann.

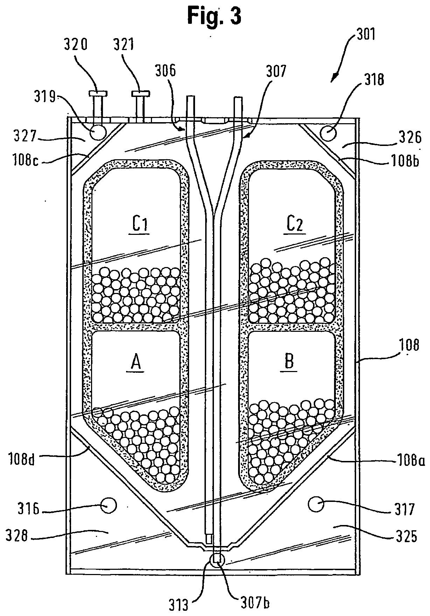

**Fig. 3** zeigt eine erfindungsgemäße Ausführungsform, die auf der Ausführungsform der **Fig. 1** aufbaut. Zusätzlich zu der Ausgestaltung von **Fig. 1** sind Durchgangspassagen 316, 317, 318 und 319 dargestellt. Die Durchgangspassagen befinden sich in Bereichen 325, 326, 327, 328 des inneren Containers oder inneren Beutels, die für Flüssigkeiten nicht zugänglich sind. Diese Bereiche stellen in der beispielhaften Darstellung durch die permanenten Schweißlinien 108, 108a, 108b, 108c, 108d abgeschweißte Bereiche dar, die keinen Flüssigkeitszugang haben. Auf alle weiteren Einzelheiten der Ausführungen nach **Fig. 1** und **Fig. 2** wird hiermit explizit verwiesen. Zunächst wird, wie voran beschrieben, der innere Beutel mit den Konzentratkammern A, B, C1, C2 befüllt und eine gebrauchsfertige Lösung hergestellt. Für den Gebrauch wird die Flüssigkeit des inneren Beutels über den Port 306 entnommen, verwendet und die gebrauchte Flüssigkeit über den Port 307, das Portende 307b und die Ausnehmung 313 in die Kammern des äußeren Beutels gefüllt. Die Durchgangspassagen 316, 317, 318 und 319 ermöglichen nun bei der Rückgabe der gebrauchten Flüssigkeit in die Kammern des äußeren Beutels eine Strömungsverbindung der Kammern. Dabei gewährleisten die unteren Durchgangspassagen 316 und 317 eine Strömungsverbindung der zuströmenden Flüssigkeit. Die oberen Durchgangsöffnungen 319 und 318 bewirken zunächst einen pneumatischen Ausgleich der durch die zuströmende Flüssigkeit verdrängten Luft. Restliche Luft wird durch die zwei Ports 320 und 321 aus dem Beutelsystem verdrängt. Durch die Durchgangspassagen 319 und 318 besteht ein Druckausgleich zwischen den beiden Kammern des äußeren Beutels, so dass nur ein Port für den Auslass der Luft aus dem äußeren Beutel notwendig ist. Die Ports sind mit Hydrophobfiltern ausgestattet, so dass die Flüssigkeit bei Benetzung der Filter gehindert ist, aus dem Beutelsystem auszutreten. Bei zunehmender Befüllung der äußeren Kammern mit gebrauchter Flüssigkeit können die Durchgangsöffnungen 319 und 318 auch dem Strömungsausgleich der Flüssigkeit dienen.

Sollte die Ausnehmung 313 durch z.B. Faltenbildung teilweise verschlossen sein und sich eine Kammer stärker befüllen als die zweite Kammer, so wird, sobald der Fluidlevel in dieser Kammer eine Durchgangspassage 316, 317, 318 oder 319 erreicht, ein Flüssigkeitsaustausch mit der weiteren Kammer des äußeren Beutels bis zum hydrostatischen Gleichgewicht stattfinden. Damit wird eine gleichmäßige Befüllung beider den inneren Beutel umgebenden Kammern erreicht.

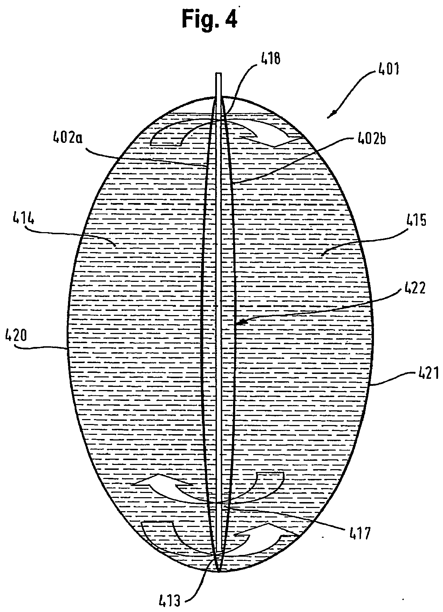

**Fig. 4** zeigt eine Ausführung des erfindungsgemäßen Beutels in einer seitlichen Querschnittsansicht. Die Abbildung zeigt in diesem Fall den bereits befüllten inneren Beutel 422, der die gebrauchsfertige Flüssigkeit enthält. In den Kammern des äußeren Beutels 414, 415 befindet sich bereits gebrauchte Flüssigkeit. Der innere Beutel 422 trennt dabei die Kammern des äußeren Beutels 420, 421 voneinander ab. Weiterhin sind Durchgangspassagen 317 und 318 gezeigt. Durch die eingezeichneten Pfeile ist ein Überströmen der gebrauchten Flüssigkeit von einer Kammer in die andere Kammer des äußeren Beutels gezeigt. Wie schematisch dargestellt, sind die Kammern 414, 415 durch die Möglichkeit des Überströmens gleichmäßig befüllt. Somit kann eine unzulässige Materialbeanspruchung bei einseitiger Kammerfüllung des äußeren Beutels vermieden werden.

## Patentansprüche

1. Containersystem für die Herstellung, Bereitstellung oder Aufnahme von medizinischen Flüssigkeiten umfassend
• einen inneren Container (422) und einen den inneren Container (422) umgebenden äußeren Container (420, 421)
• Zugänge (306, 307) an dem inneren (422) und dem äußeren Container (420, 421), zur Befüllung und/oder Entnahme von Flüssigkeiten,
**dadurch gekennzeichnet**
**dass** das Containersystem des weiteren umfasst:
• ein oder mehrere Durchgangsöffnungen (316, 317, 318, 319) durch den inneren Container (422), die so ausgebildet sind, dass Teile des durch den äußeren Container (420, 421) umgrenzten Volumens miteinander in Strömungsverbindung gebracht werden.

2. Containersystem nach Anspruch 1, **dadurch gekennzeichnet, dass** es sich bei den medizinischen Flüssigkeiten um Flüssigkeiten für die extrakorporale Blutbehandlung und/oder der Nierentherapie handelt.

3. Containersystem nach Anspruch 1 oder 2, **dadurch gekennzeichnet, dass** der innere Container (422) ein Beutel ist.

4. Containersystem nach einem der vorhergehenden Ansprüche, **dadurch gekennzeichnet, dass** der äußere Container (420, 421) ein Beutel ist.

5. Containersystem nach einem oder mehreren der Ansprüche 1 bis 4, **dadurch gekennzeichnet, dass** die Durchgangsöffnungen (316, 317, 318, 319) bei hängender oder aufrechter Lagerung des Containersystems im unteren Bereich und / oder im oberen Bereich des Containersystems angeordnet sind.

6. Containersystem nach einem oder mehreren der Ansprüche 1 bis 5, **dadurch gekennzeichnet, dass** der innere Container (422) und / oder der äußere Container (420, 421) mit Filtern versehen sind, die eine keimarme Entlüftung des Beutels bei Befüllung und/oder Flüssigkeitsentnahme gewährleisten.

7. Containersystem nach einem oder mehreren der Ansprüche 1 bis 6, **dadurch gekennzeichnet, dass** das Containersystem aus wenigstens vier übereinander angeordneten Folien (202a, 202b) aufgebaut ist, die eine umfängliche gemeinsame Begrenzungslinie, vorzugsweise Schweißnaht (108) beinhalten, so dass eine innere Kammer (417), entsprechend dem Volumen des inneren Beutels (422) und zwei weitere Kammern (214, 215, 414, 415), entsprechend dem Volumen des äußeren Beutels (420, 421) gebildet werden.

8. Containersystem nach Anspruch 7, **dadurch gekennzeichnet, dass** eine der Kammern (417) des inneren (422) und / oder äußeren Beutels ein oder mehrere Kompartimente (A, B, C1, C2) enthält, die mit Konzentraten befüllt sind.

9. Containersystem nach Anspruch 8, **dadurch gekennzeichnet, dass** ein oder mehrere Kompartimente (A, B, C1, C2) der Kammern (417) im inneren (422) und/oder äußeren Beutel lösbare Begrenzungsmittel, insbesondere Peelnähte (209c, 209d) aufweisen.

10. Containersystem nach Anspruch 8 oder 9, **dadurch gekennzeichnet, dass** die Kompartimente (A, B, C1, C2) pulverförmige, im wässrigen lösliche Substanzen enthalten.

11. Containersystem nach einem oder mehreren der vorangehenden Ansprüche, **dadurch gekennzeichnet, dass** das Containersystem ein Flüssigkeitsvolumen von 5 bis 120 Litern umfasst.

12. Containersystem nach einem oder mehreren der vorangehenden Ansprüche, **dadurch gekennzeichnet, dass** das Containersystem ein Flüssigkeitsvolumen von 15 bis 90 Litern umfasst.

13. Containersystem nach einem oder mehreren der vorangehenden Ansprüche, **dadurch gekennzeichnet, dass** das Containersystem ein Flüssigkeitsvolumen von 40 bis 80 Litern umfasst.

14. Containersystem nach einem oder mehreren der vorangehenden Ansprüche 3 bis 13, **dadurch gekennzeichnet, dass** der oder die Beutel aus einem elastisch dehnbaren Folienmaterial (202a, 202b) bestehen.

## Claims

1. A container system for producing, supplying or holding medical liquids comprising
▪ an inner container (422) and an outer container (420, 421) surrounding the inner container (422),
▪ ports (306, 307) at the inner container (422) and the outer container (420, 421) for filling and/or removing liquids,
**characterized in that**
the container system furthermore comprises:
▪ one or more through-openings (316, 317, 318, 319) through the inner container (422) that are configured such that parts of the volume defined by the outer container (420, 421) are brought into flow communication with one.

2. A container system according to claim 1, **characterized in that** the medical liquids are liquids for the extracorporeal blood treatment and/or renal therapy.

3. A container system according to claim 1 or claim 2, **characterized in that** the inner container (422) is a bag.

4. A container system according to any one of the preceding claims, **characterized in that** the outer container (420, 412) is a bag.

5. A container system according to one or more of claims 1 to 4, **characterized in that** the through-openings (316, 317, 318, 319) are arranged in a lower area and/or an upper area of the container system when the container system is supported upright or in a suspended position.

6. A container system according to one or more of claims 1 to 5, **characterized in that** the inner container (422) and/or the outer container (420, 421) is/are provided with filters which ensure germ-free venting of the bag during filling and/or withdrawal of liquid.

7. A container system according to one or more of claims 1 to 6, **characterized in that** the container system is constructed of at least four films (202a, 202b) arranged one above the other, having a circumferential common bordering line, preferably a weld seam (108), so that an inner chamber (417) corresponding to the volume of the inner bag (422) and two further chambers (214, 215, 414, 415) corresponding to the volume of the outer bag (420, 421) are formed.

8. A container system according to claim 7, **characterized in that** one of the chambers (417) of the inner bag (422) and/or the outer bag contains one or more compartments (A, B, C1, C2) filled with concentrates.

9. A container system according to claim 8, **characterized in that** one or more compartments (A, B, C1, C2) of the chambers (417) has/have bordering means, in particular peel seams (209c, 209d), which can be released in the inner bag (422) and/or the outer bag.

10. A container system according to claim 8 or 9, **characterized in that** the compartments (A, B, C1, C2) contain powdered water-soluble substances.

11. A container system according to one or more of the preceding claims, **characterized in that** the container system has a liquid volume of 5 to 120 liters.

12. A container system according to one or more of the preceding claims, **characterized in that** the container system has a liquid volume of 15 to 90 liters.

13. A container system according to one or more of the preceding claims, **characterized in that** the container system has a liquid volume of 40 to 80 liters.

14. A container system according to one or more of the preceding claims 3 to 13, **characterized in that** the bag(s) is/are made of an elastically extensible film material (202a, 202b).

## Revendications

1. Système de contenants destiné à la fabrication, la mise à disposition ou la réception de liquides médicaux, comprenant
• un contenant intérieur (422) et un contenant extérieur (420, 421) entourant le contenant intérieur (422)
• des accès (306, 307) au contenant intérieur (422) et extérieur (420, 421), pour le remplissage et/ou le prélèvement de liquides,
**caractérisé en ce que**
le système de contenants comprend en outre :
• un ou plusieurs orifices de passage (316, 317, 318, 319) à travers le contenant intérieur (422), qui sont conçus de telle manière que des parties du volume délimité par le contenant extérieur (420, 421) sont amenées en liaison d'écoulement les unes avec les autres.

2. Système de contenants selon la revendication 1, **caractérisé en ce que** les liquides médicaux sont des liquides destinés au traitement extracorporel du sang et/ou à la thérapie rénale.

3. Système de contenants selon la revendication 1 ou 2, **caractérisé en ce que** le contenant intérieur (422) est une poche.

4. Système de contenants selon l'une des revendications précédentes, **caractérisé en ce que** le contenant extérieur (420, 421) est une poche.

5. Système de contenants selon une ou plusieurs des revendications 1 à 4, **caractérisé en ce que** les orifices de passage (316, 317, 318, 319) sont disposés dans la partie inférieure et/ou dans la partie supérieure du système de contenants lors d'un stockage suspendu ou vertical du système de contenant.

6. Système de contenants selon une ou plusieurs des revendications 1 à 5, **caractérisé en ce que** le contenant intérieur (422) et/ou le contenant extérieur (420, 421) sont pourvus de filtres, qui garantissent une évacuation de l'air pauvre en germes lors du remplissage et/ou du prélèvement de liquide.

7. Système de contenants selon une ou plusieurs des revendications 1 à 6, **caractérisé en ce que** le système de contenants est constitué d'au moins quatre feuilles (202a, 202b) disposées les unes sur les autres, qui comportent une ligne de délimitation commune périphérique, de préférence un cordon de soudure (108), de sorte qu'une chambre intérieure (417), correspondant au volume de la poche intérieure (422), et deux autres chambres (214, 215, 414, 415), correspondant au volume de la poche extérieure (420, 421), sont formées.

8. Système de contenants selon la revendication 7, **caractérisé en ce qu'**une des chambres (417) de la poche intérieure (422) et/ou extérieure contient un ou plusieurs compartiments (A, B, C1, C2), qui sont remplis de concentrés.

9. Système de contenants selon la revendication 8, **caractérisé en ce qu'**un ou plusieurs compartiments (A, B, C1, C2) des chambres (417) dans la poche intérieure (422) et/ou extérieure comportent des moyens de délimitation détachables, en particulier des soudures pelables (209c, 209d).

10. Système de contenants selon la revendication 8 ou 9, **caractérisé en ce que** les compartiments (A, B, C1, C2) contiennent des substances poudreuses, solubles en milieu aqueux.

11. Système de contenants selon une ou plusieurs des revendications précédentes, **caractérisé en ce que** le système de contenants comprend un volume de liquide de 5 à 120 litres.

12. Système de contenants selon une ou plusieurs des revendications précédentes, **caractérisé en ce que** le système de contenants comprend un volume de liquide de 15 à 90 litres.

13. Système de contenants selon une ou plusieurs des revendications précédentes, **caractérisé en ce que** le système de contenants comprend un volume de liquide de 40 à 80 litres.

14. Système de contenants selon une ou plusieurs des revendications précédentes 3 à 13, **caractérisé en ce que** la ou les poches se composent d'un matériau en feuille (202a, 202b) extensible de manière élastique.
